# EUROPÄISCHE PATENTSCHRIFT

(11) **EP 2 024 340 B1**
(45) Veröffentlichungstag und Bekanntmachung des Hinweises auf die Patenterteilung: **18.07.2012**
(21) Anmeldenummer: 07724731.0
(22) Anmeldetag: 30.04.2007
(51) Int. Cl.: C07D 239/42, A61K 31/505, A61K 31/506, A61P 3/08

(54) **4,5-DIPHENYL-PYRIMIDINYL-AMINO SUBSTITUIERTE CARBONSÄUREN, VERFAHREN ZU IHRER HERSTELLUNG UND IHRE VERWENDUNG ALS ARZNEIMITTEL**
4,5-DIPHENYL-PYRIMIDINYL-AMINO SUBSTITUTED CARBOXYLIC ACIDS, METHOD FOR THE PRODUCTION AND USE THEREOF AS MEDICAMENTS
ACIDES CARBOXYLIQUES SUBSTITUÉS 4,5-DIPHÉNYL-PYRIMIDINYL-AMINO, PROCÉDÉ DE PRODUCTION ASSOCIÉ ET LEUR UTILISATION COMME MÉDICAMENTS

(30) Priorität: 11.05.2006 DE 102006021874
(43) Veröffentlichungstag der Anmeldung: 18.02.2009
(73) Patentinhaber: SANOFI, 75008 Paris (FR)
(72) Erfinder: DEFOSSA, Elisabeth, 65926 Frankfurt am Main (DE); GOERLITZER, Jochen, 65926 Frankfurt am Main (DE); KLABUNDE, Thomas, 65926 Frankfurt am Main (DE); DROSOU, Viktoria, 65926 Frankfurt am Main (DE); STENGELIN, Siegfried, 65926 Frankfurt am Main (DE); HASCHKE, Guido, 65926 Frankfurt am Main (DE); HERLING, Andreas, 65926 Frankfurt am Main (DE); BARTOSCHEK, Stefan, 65926 Frankfurt am Main (DE)
(74) Vertreter: Fischer, Hans-Jürgen
(86) Internationale Anmeldenummer: PCT/EP2007/003804
(87) Internationale Veröffentlichungsnummer: WO 2007/131620

(56) Entgegenhaltungen:
- WO-A-2005/103022
- WO-A-2006/018280

## Beschreibung

Die Erfindung betrifft 4,5-Diphenyl-pyrimidinyl-amino substituierte Carbonsäuren sowie deren physiologisch verträgliche Salze.

Es sind bereits strukturähnliche Verbindungen im Stand der Technik beschrieben (siehe WO 2005/103022).

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Hyperglykämie und von Diabetes geeignet sind.

Die Erfindung betrifft daher Verbindungen der Formel I, 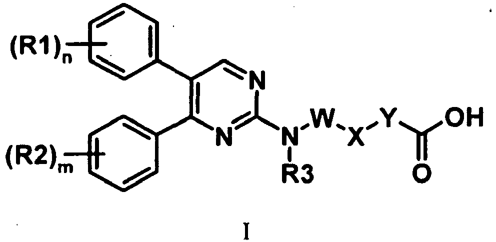 worin bedeuten
- R1, R2: unabhängig voneinander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkyl ein oder mehrfach mit F, Cl, Br oder CN substituiert sein kann;
- n: 0, 1, 2, 3, 4 oder 5;
- m: 0, 1, 2, 3, 4 oder 5;
- W: Bindung, (C₁-C₇)-Alkylen, (C₂-C₇)-Alkenylen oder (C₂-C₇)-Alkinylen, wobei Alkylen, Alkenylen und Alkinylen ein oder mehrfach mit R4 substituiert sein können;
- X: Bindung, mono-, bi- oder tricyclischer (C₃-C₁₂)-Cycloalkylring, wobei der Cycloalkylring ein oder mehrfach mit R5 substituiert sein kann;
- Y: Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
- R3: H oder (C₁-C₆)-Alkyl;
- R4: NH₂, NH(C₁-C₄)-Alkyl, N[(C₁-C₄)-Alkyl]₂, F, Cl, Br, CN, OH, O-(C₁-C₆)-Alkyl, (C₁-C₆)-Alkyl, (C₂-C₆)-Alkenyl oder (C₂-C₆)-Alkinyl, wobei Alkl, Alkenyl und Alkinyl ein oder mehrfach mit F, Cl, Br oder CN substituiert sein können;
- R5: F, Cl, Br, CN, (C₁-C₄)-Alkyl oder O-(C₁-C₄)-Alkyl, wobei Alkl ein oder mehrfach mit F, Cl, Br oder CN substituiert sein kann;
wobei W, X und Y nicht gleichzeitig die Bedeutung Bindung besitzen und deren physiologisch verträgliche Salze.

Bevorzugt sind Verbindungen der Formel I, worin ein oder mehrere Reste die folgenden Bedeutungen haben:
- R1, R2: unabhängig voneinander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkyl ein oder mehrfach mit F, Cl, Br oder CN substituiert sein kann;
- n: 0, 1, 2, 3, 4 oder 5;
- m: 0, 1, 2, 3, 4 oder 5;
- W: Bindung, (C₁-C₇)-Alkylen, (C₂-C₇)-Alkenylen oder (C₂-C₇)-Alkinylen, wobei Alkylen, Alkenylen und Alkinylen ein oder mehrfach mit R4 substituiert sein können;
- X: Bindung, mono-, bi- oder tricyclischer (C₃-C₁₂)-Cycloalkylring, wobei der Cycloalkylring ein oder mehrfach mit R5 substituiert sein kann;
- Y: Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
- R3: H oder (C₁-C₆)-Alkyl;
- R4: NH₂, NH(C₁-C₄)-Alkyl, N[(C₁-C₄)-Alkyl]₂;
- R5: F, Cl, Br, CN, (C₁-C₄)-Alkyl oder O-(C₁-C₄)-Alkyl, wobei Alkl ein oder mehrfach mit F, Cl, Br oder CN substituiert sein kann;
wobei W und X nicht gleichzeitig die Bedeutung Bindung besitzen;
und deren physiologisch verträgliche Salze.

Besonders bevorzugt sind Verbindungen der Formel I, worin ein oder mehrere Reste die folgenden Bedeutungen haben:
- R1, R2: unabhängig von einander (C₁-C₆)-Alkyl, O-(C₁-C₆)-Alkyl, OH, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkyl ein oder mehrfach mit F, Cl, Br oder CN substituiert sein kann;
- n: 0, 1, 2;
- m: 0, 1, 2;
- W: Bindung, (C₁-C₇)-Alkylen, (C₂-C₇)-Alkenylen oder (C₂-C₇)-Alkinylen, wobei Alkylen, Alkenylen und Alkinylen ein oder mehrfach mit R4 substituiert sein können;
- X: Bindung, mono-, bi- oder tricyclischer (C₃-C₁₂)-Cycloalkylring, wobei der Cycloalkylring ein oder mehrfach mit R5 substituiert sein kann;
- Y: Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
- R3: H oder (C₁-C₆)-Alkyl;
- R4: NH₂, NH(C₁-C₄)-Alkyl, N[(C₁-C₄)-Alkyl]₂;
- R5: F, Cl, Br, CN, (C₁-C₄)-Alkyl oder O-(C₁-C₄)-Alkyl;
wobei W und X nicht gleichzeitig die Bedeutung Bindung besitzen;
und deren physiologisch verträgliche Salze.

Ganz besonders bevorzugt sind Verbindungen der Formel I, worin ein oder mehrere Reste die folgenden Bedeutungen haben:
- R1, R2: unabhängig voneinander (C₁-C₆)-Alkyl, O-(C₁-C₆)-Alkyl, OH, CN, F, Cl, Br, O-Phenyl, CF₃, OCF₃ oder OCH₃, wobei Alkyl ein oder mehrfach mit F, Cl, Br oder CN substituiert sein kann;
- n: 0;
- m: 0;
- W: Bindung, (C₁-C₇)-Alkylen; oder falls X eine Bindung ist W die Bedeutung (C₄-C₇)-Alkylen besitzt;
- X: Bindung, mono- oder bicyclischer (C₃-C₁₂)-Cycloalkylring, wobei der Cycloalkylring ein oder mehrfach mit R5 substituiert sein kann;
- Y: Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
- R3: H oder (C₁-C₆)-Alkyl;
- R4: NH₂, NH(C₁-C₄)-Alkyl, N[(C₁-C₄)-Alkyl]₂;
- R5: F, Cl, Br, CN, (C₁-C₄)-Alkyl oder O-(C₁-C₄)-Alkyl;
wobei W und X nicht gleichzeitig die Bedeutung Bindung besitzen;
und deren physiologisch verträgliche Salze.

Können Reste oder Substituenten mehrfach in den Verbindungen der Formeln I auftreten, so können sie alle unabhängig voneinander die angegebene Bedeutung haben und gleich oder verschieden sein.

Die Alkyl-, Alkenyl-, Alkinyl-, Alkylen-, Alkenylen- und Alkinylenreste in den Resten W, Y R1, R2, R3, R4 und R5 können sowohl geradkettig wie verzweigt sein.

Die Erfindung betrifft Verbindungen der Formel 1 in Form ihrer Salze, Racemate, racemischen Mischungen und reinen Enantiomere, und deren Diastereomere und Mischungen davon.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon- und Weinsäure. Für medizinische Zwecke wird in besonders bevorzugter Weise das Chlorsalz verwendet. Geeignete physiologisch verträgliche basische Salze sind Ammoniumsalze, Alkalimetallsalze (wie Natrium- und Kaliumsalze), Erdalkalisalze (wie Magnesium- und Calciumsalze), Zinksalze, Trometamol- (2-Amino-2-hydroxymethyl-1,3-propandiol), Diethanolamin-, Lysin-, Arginin-, Cholin-, Meglumin- oder Ethylendiamin-Salze.

Salze mit einem nicht physiologisch verträglichen Anion oder Kation gehören ebenfalls in den Rahmen der Erfindung als nützliche Zwischenprodukte für die Herstellung oder Reinigung physiologisch verträglicher Salze und/oder für die Verwendung in nicht-therapeutischen, zum Beispiel in-vitro-Anwendungen.

Beschrieben wesden zudem Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formeln.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Verbindungen der Formel I und deren physiologisch verträgliche Salze stellen ideale Arzneimittel zur Behandlung von erhöhten Lipidkonzentrationen im Blut, dem metabolischen Syndrom, Diabetes, Insulinresistenz, der Dysregulation von LDL, HLD und VLDL oder Herzkreislauferkrankungen und Lipidstoffwechselstörungen, insbesondere von Hyperlipidämie dar.

Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln können beispielsweise von 0,01 bis 100 mg, typischerweise von 0,02 bis 50 mg, enthalten. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05 % bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

### Kombinationen mit anderen Medikamenten

Die erfindungsgemäßen Verbindungen können alleine oder in Kombination mit einem oder mehreren weiteren pharmakologischen Wirkstoffen, die beispielsweise günstige Wirkungen auf Stoffwechselstörungen bzw. häufig damit assoziierten Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet:
Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871 oder WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe, Biguanidine,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4), . Inhibitoren der Glutamin-Fructose-6-Phosphat-Amidotransferase (GFAT), GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11β-HSD1,
Inhibitoren der Protein-Tyrosin-Phosphatase- 1B (PTP 1 B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.), WO2005062824 (Merck & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483 oder CS-011 (Rivoglitazon), verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030, AVE 8042, AVE 8134, AVE 0847 oder wie in PCT/US 00/11833, PCT/US 00/11490, DE10142734.4 oder in J.P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516, verabreicht.

Bei einer Ausführungsform der Erfindung wird die Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I in Kombination mit einem Fibrat, wie z.B. Fenofibrat, Clofibrat oder Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705, verabreicht.

Bei einer Ausführungsform der Erfindung wird die Verbindung der Formel 1 in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586 oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, β-Caroten oder Selen, verabreicht.

Bei einer Ausführungsform der Erfindung wird die Verbindung der Formel I in Kombination mit einem Vitamin, wie z. B. Vitamin B6 oder Vitamin B12, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) Antagonist, wie z.B. Gemcabene (CI-1027), verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform der Erfindung wird die Verbindung der Formel I in Kombination mit einem Sulfonylharnstoff, wie z.B. Tolbutamid, Glibenclamid, Glipizid oder Glimepirid, verabreicht.

Bei einer Ausführungsform der Erfindung wird die Verbindung der Formel I in Kombination mit einem Biguanid, wie z.B. Metformin, verabreicht.

Bei wieder einer Ausführungsform der Erfindung wird die Verbindung der Formel I in Kombination mit einem Meglitinid, wie z.B. Repaglinide oder Nateglinid, verabreicht.

Bei einer Ausführungsform der Erfindung wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem α-Glukosidase-Inhibitor, wie z.B. Miglitol oder Acarbose, verabreicht.

Bei einer Ausführungsform der Erfindung wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I in Kombination mit Glukagon-Rezeptor-Antagonisten, wie z.B. A-770077, NNC-25-2504 oder wie in WO2004100875 oder WO2005065680 beschrieben, verabreicht.

Bei einer Ausführungsform der Erfindung wird die Verbindung der Formel I in Kombination mit Aktivatoren der Glukokinase, wie z. B. RO-4389620, LY-2121260 (WO2004063179), PSN-105, PSN-110, GKA-50oder solchen wie sie z. B. von Prosidion in WO2004072031, W02004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I in Kombination mit einem Inhibitor der Glukoneogenese, wie z. B. FR-225654, verabreicht.

Bei einer Ausführungsform der Erfindung wird die Verbindung der Formel I in Kombination mit Inhibitoren der Fructose-1,6-bisphosphatase (FBPase), wie z.B. CS-917, verabreicht.

Bei einer Ausführungsform der Erfindung wird die Verbindung der Formel I in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform der Erfindung wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO200505890 WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform der Erfindung wird die Verbindung der Formel I in Kombination mit Hemmstoffen der 11-beta-Hydroxysteroid-Dehydrogenase-1 (11β-HSD1), wie z. B. BVT-2733 oder solche, wie sie z. B. in WO200190090-94, WO200343999, WO2004112782, WO200344000, WO200344009, WO2004112779, W02004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877 oder WO2005097759 beschrieben sind, verabreicht.

Bei einer Ausführungsform der Erfindung wird die Verbindung der Formel I in Kombination mit Inhibitoren der Protein-Tyrosin-Phosphatase-1B (PTP1B), wie sie z. B. in W0200119830-31, WO200117516, WO2004506446, WO2005012295, PCT/EP2005/005311, PCT/EP2005/005321, PCT/EP2005/007151, PCT/EP2005/ oder DE 10 2004 060542.4 beschrieben sind, verabreicht.

Bei einer Ausführungsform der Erfindung wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I in Kombination mit Inhibitoren der hormon-sensitiven Lipase (HSL), wie z. B. in WO2005073199 beschrieben, verabreicht.

Bei einer Ausführungsform der Erfindung wird die Verbindung der Formel I in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727 oder WO2004046117 beschrieben, verabreicht.

Bei einer Ausführungsform der Erfindung wird die Verbindung der Formel I in Kombination mit einem Inhibitor der Protein Kinase C beta (PKC beta), wie z. B. Ruboxistaurin, verabreicht.

Bei einer Ausführungsform der Erfindung wird die Verbindung der Formel I in Kombination mit einem Endothelin-A-Rezeptor Antagonisten, wie z. B. Avosentan (SPP-301), verabreicht.

Bei einer Ausführungsform der Erfindung wird die Verbindung der Formel I in Kombination mit Inhibitoren der "I-kappaB Kinase" (IKK Inhibitoren), wie sie z. B. in W02001000610, WO2001030774, WO2004022553 oder WO2005097129 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer weiteren Ausführungsform der Erfindung wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558); NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A); Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind; Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-3 WO200411038-39, WO2005016286, WO2005007111, WO2005007628, US20050054679, WO2005027837, WO2005028456, WO2005063761-62, WO2005061509 oder W02005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, W02005047251, EP1538159, WO2004072076 oder WO2004072077 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in
WO200064884, WO2005082893 beschrieben sind); TNF - Agonisten; CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, W02004072025, WO2005070898, WO2005070925, WO2004039780, WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356, BVT-933 oder solche, wie sie in WO200077010, WO20077001-02, WO2005019180, WO2003064423, WO200242304 oder WO2005082859 beschrieben sind);
5-HT6 Rezeptor Antagonisten, wie sie z.B. in WO2005058858 beschrieben sind; Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solche, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind);
Inhibitoren der Diacylglycerol O-Acyltransferasen (DGATs) wie z. B. in US2004/0224997, WO2004094618, WO200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron
TR-β - Agonisten
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421 oder WO2005092316 beschrieben, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei noch einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

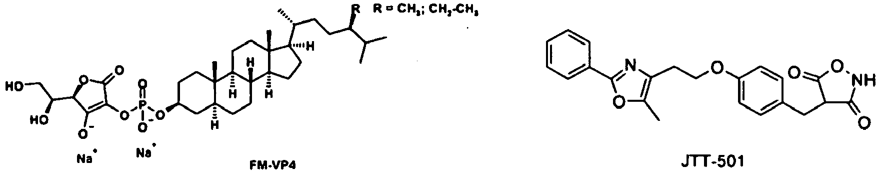

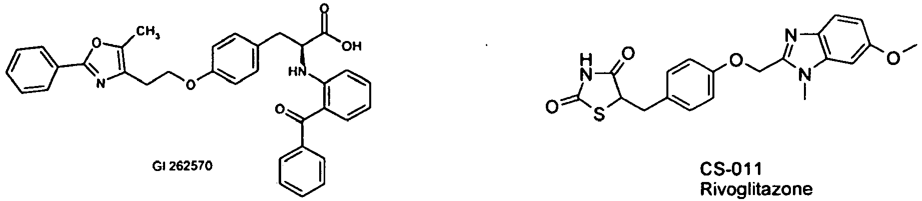

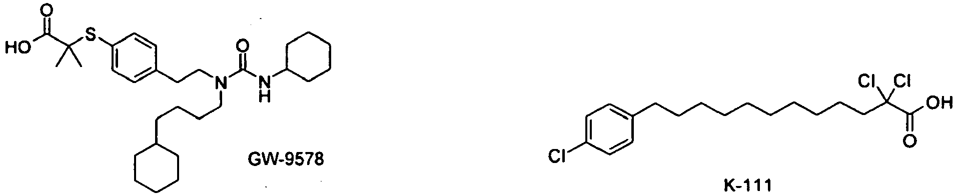

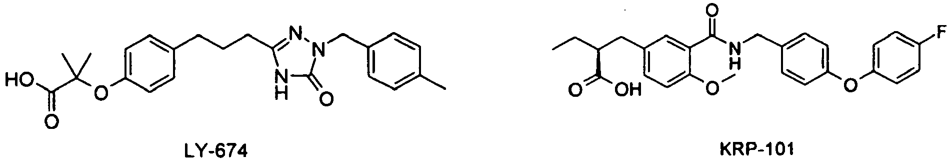

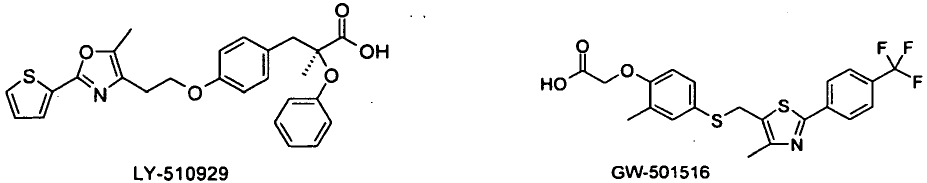

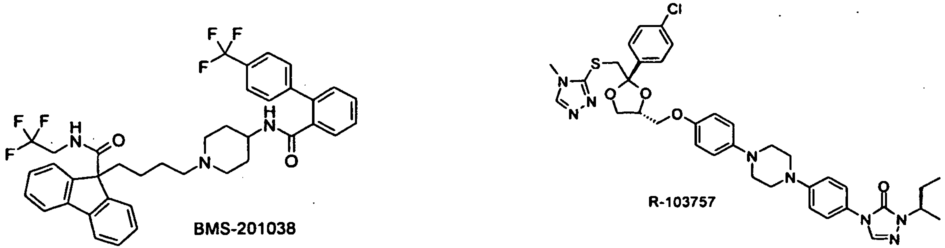

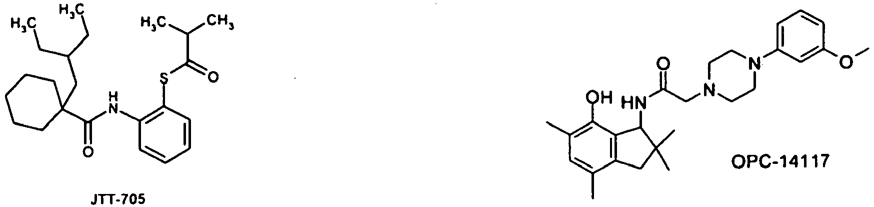

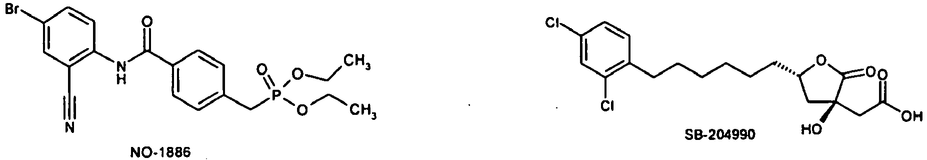

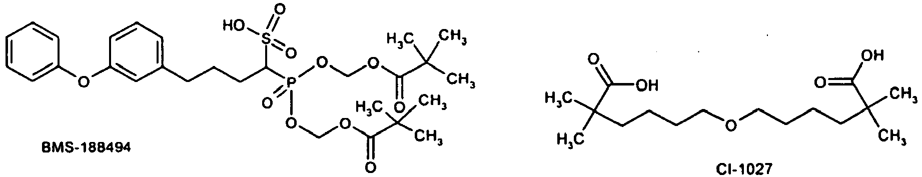

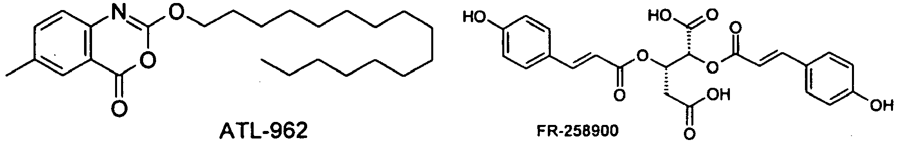

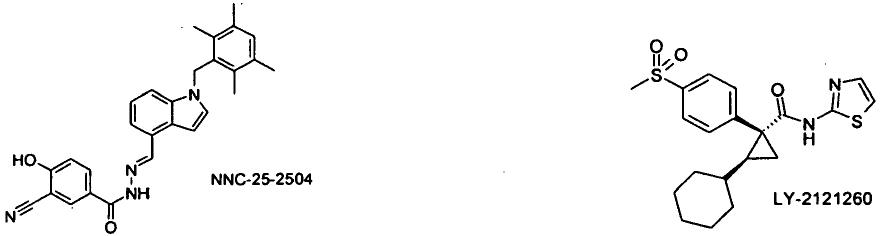

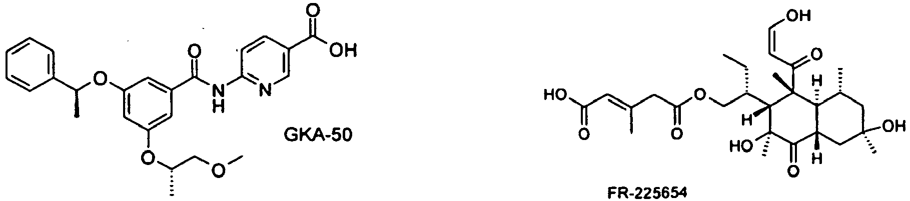

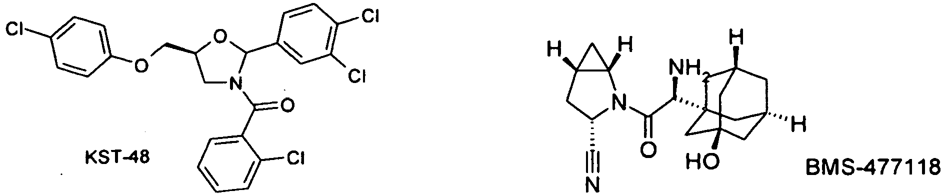

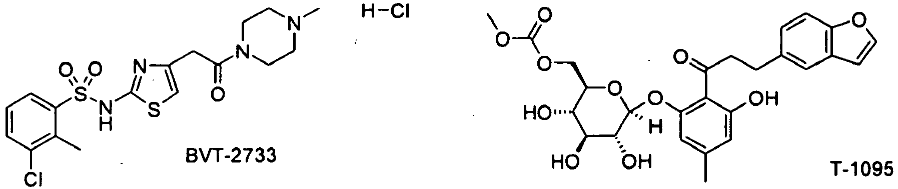

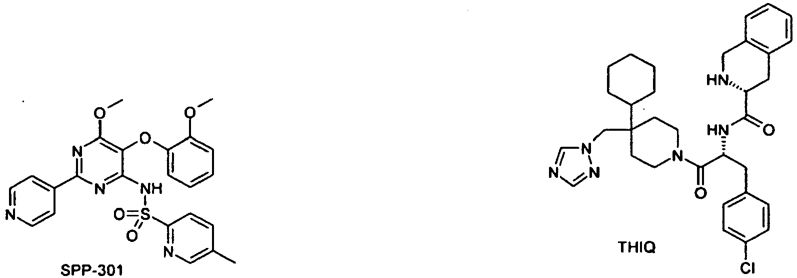

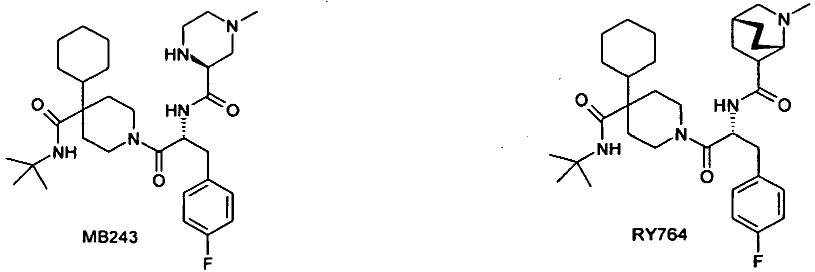

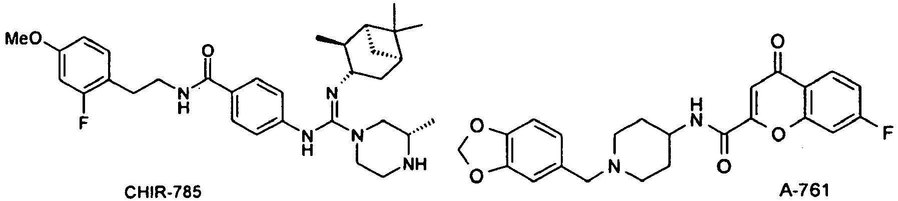

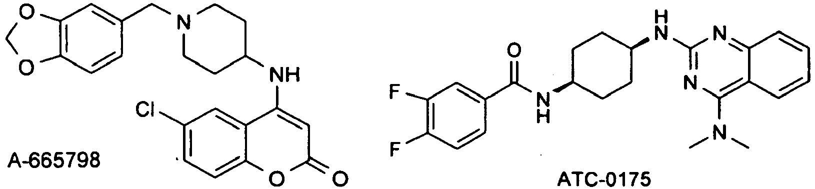

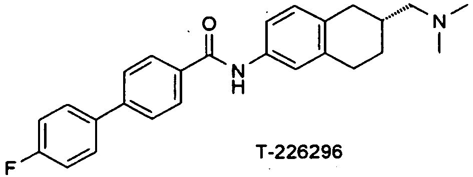

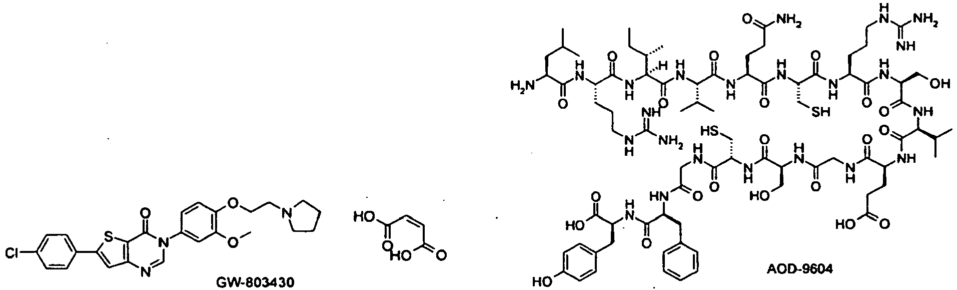

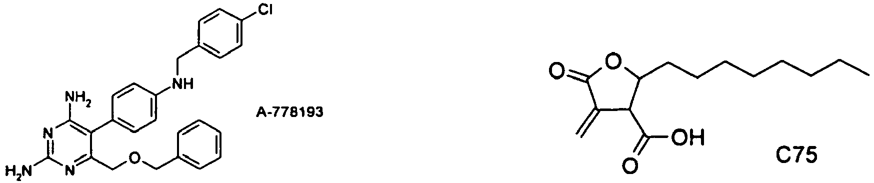

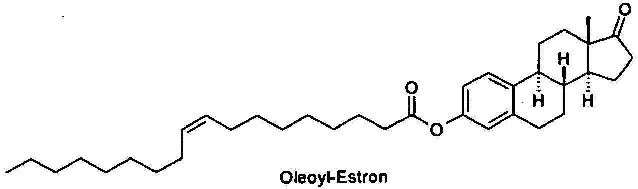

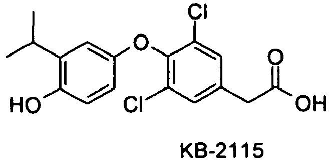

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Die nachfolgend aufgeführten Beispiele dienen zur Erläuterung der Erfindung, ohne diese jedoch einzuschränken.

**Tabelle 1:**

| | | | | |
|---|---|---|---|---|
| 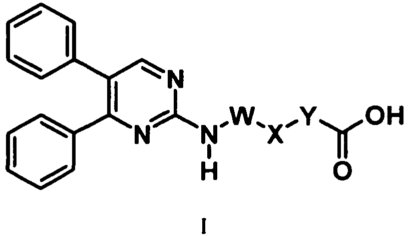 | | | | |

| Bsp. | W | X | Y | Salz |
|---|---|---|---|---|
| 1 | -CH₂- | 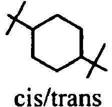 | - | |
| 2 | - | 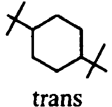 | - | |
| 3 | -(CH₂)₇- | - | - | TFA |
| 4 | -(CH₂)₆- | - | - | TFA |
| 5 | -(CH₂)₅- | - | - | TFA |
| 6 | -(CH₂)₄- | - | -CH(NH₂)-CH₂-, R | TFA |
| 7 | - | 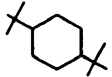 | -CH₂-CH(NH₂)-, S | TFA |

Die Wirksamkeit der Verbindungen wurde wie folgt getestet:

In-vitro FLIPR-Assay mit rekombinanten Zellen, die den GPCR GPR40 exprimieren Funktionsüberprüfende Assays wurden mittels der FLIPR-Technik ("Fluorescence Imaging Plate Reader", Molecular Devices Corp.) durchgeführt. Hierzu wurden agonist-induzierte Änderungen der intrazellulären Konzentration von Ca²⁺ in rekombinanten HEK293 Zellen bestimmt, die den GPCR GPR40 exprimierten.

Für die Untersuchungen wurden Zellen in 96-well-Mikrotiterplatten (60000 Zellen/well) ausgesät und über Nacht wachsen gelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz zugegeben und Änderungen in der intrazellulären Ca²⁺-Konzentration im FLIPR-Gerät gemessen. Ergebnisse wurden als prozentuale Änderung relativ zur Kontrolle dargestellt (0 %: keine Testsubstanz addiert; 100 %: 10 µM Referenzagonist Linolsäure addiert).

**Tabelle 2: Biologische Aktivität**

| Bsp. | % Aktivierung @ 100 µM |
|---|---|
| 1 | 79 |
| 3 | 104 |
| 4 | 94 |
| 5 | 73 |

Aus der Tabelle ist abzulesen, dass die Verbindungen der Formel I den GPR40 Rezeptor aktivieren und dadurch gut zur Behandlung von Hyperglykämie und von Diabetes geeignet sind. Durch die Verbindungen der Formel I wird die Insulinausschüttung erhöht (siehe Itoh et al., Nature 2003, 422, 173-176).

Die Verbindungen der Formel I können auch eine entsprechende Wirkung auf den GPR120 Rezeptor zeigen.

Die Verbindungen der Formel I lassen sich dadurch herstellen, dass man geeignete Ausgangsstoffe der Formel II 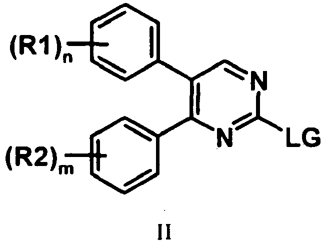 wobei LG eine Austrittsgruppe wie zum Beispiel Halogen, O-Mesylat, O-Tosylat, O-Triflat oder SO₂Me bedeuten kann und R1 und R2 die oben genannten Bedeutungen haben, mit Verbindungen der Formel III, 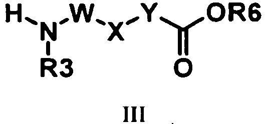 wobei OR6 eine geeignete Schutzgruppe für Ester darstellt und R3, W, X und Y die oben genannten Bedeutungen haben, unter Einfluss geeigneter anorganischer und organischer Basen wie zum Beispiel Na₂CO₃, Cs₂CO₃, K₂CO₃, NaH, DBU, DBN oder Kalium-tert-butylat in einem geeigneten Lösungsmittel wie zum Beispiel Dimethylformamid, Dimethylsulfoxid, Toluol oder Tetrahydrofuran zu Verbindungen der Formel IV umsetzt.

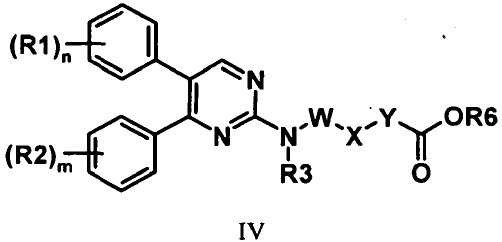

Wenn die Esterschutzgruppe unter den Reaktionsbedingungen nicht abgespalten wurde, wird anschließend die Esterschutzgruppe nach Literatur-bekannten Verfahren abgespalten und man erhält die Verbindungen der Formel I.

Nachfolgend wird die Herstellung der Beispiele detailliert beschrieben:

### Experimenteller Teil:

### Beispiel 1

### 4-[(4,5-Diphenyl-pyrimidin-2-ylamino)-methyl]-cyclohexancarbonsäure

Zu einer Suspension von 133 mg (0,84 mmol) 4-Aminomethyl-cyclohexancarbonsäure in 5 ml Toluol gab man 550 mg (1,69 mmol) Cäsiumcarbonat und rührte bei Raumtemperatur. Nach 30 Minuten wurden 150 mg (0,56 mmol) 2-Chlor-4,5-diphenyl-pyrimidin zugegeben und 31 Stunden bei 120 °C gerührt. Das Lösungsmittel wurde im Vakuum entfernt und der Rückstand mit Dichlormethan und Wasser versetzt. Der entstandene Niederschlag wurde abgesaugt. Man erhielt 135 mg (62 %) des gewünschten Produktes.
MS: m/z = 388,5 (M+H).

### Beispiel 2

### trans-4-(4,5-Diphenyl-pyrimidin-2-ylamino)-cyclohexancarbonsäure

### trans-4-(4,5-Diphenyl-pyrimidin-2-ylamino)-cyclohexancarbonsäureethylester

Zu einer Suspension von 175 mg (0,84 mmol) trans-4-Amino-cyclohexancarbonsäureethylester in 5 ml Toluol gab man 550 mg (1,69 mmol) Cäsiumcarbonat und rührte bei Raumtemperatur. Nach 30 Minuten wurden 150 mg (0,56 mmol) 2-Chlor-4,5-diphenyl-pyrimidin zugegeben und 4 Tage bei 120 °C gerührt. Das Lösungsmittel wurde im Vakuum entfernt und der Rückstand zwischen Dichlormethan und Wasser verteilt. Die organische Phase wurde getrocknet und im Hochvakuum eingeengt. Der Rückstand wurde versucht über eine Kieselgelsäule zu reinigen (Heptan/Isopropanol, Gradient 100 % auf 5 % in 15 Minuten). Man erhielt eine Mischfraktion aus der das Produkt auskristallisierte. Der Niederschlag wurde abgesaugt. So erhielt man 78 mg (34 %) des gewünschten Produktes.
MS: *m*/*z* = 402,5 (M+H).
trans-4-(4,5-Diphenyl-pyrimidin-2-ylamino)-cyclohexancarbonsäure 70 mg (0,17 mmol) trans-4-(4,5-Diphenyl-pyrimidin-2-ylamino)-cyclohexancarbonsäureethylester wurden mit 872 µl Tetrahydrofuran und 872 µl 1 n wässriger Lithiumhydroxidlösung versetzt und 48 Stunden bei Raumtemperatur gerührt. Das Reaktionsgemisch wurde mit Wasser verdünnt, mit Schwefelsäure angesäuert und der entstandenen Niederschlag wurde abgesaugt. Man erhielt 64 mg (98 %) des gewünschten Produktes.
MS: m/z = 374,5 (M+H).

### Beispiel 3

### 8-(4,5-Diphenyl-pyrimidin-2-ylamino)-oktansäure Trifluoracetat

### 8-(4,5-Diphenyl-pyrimidin-2-ylamino)-oktansäureethylester Trifluoracetat

Zu einer Lösung von 439 mg (1,69 mmol) 8-Amino-oktansäureethylester in 10 ml Dimethylformamid gab man 1,83 g (5,63 mmol) Cäsiumcarbonat und rührte bei Raumtemperatur. Nach 30 Minuten wurde eine Lösung von 300 mg (1,13 mmol) 2-Chlor-4,5-diphenyl-pyrimidin in 5 ml Dimethylformamid zugegeben und 4 Stunden bei 90 °C gerührt. Das Lösungsmittel wurde eingeengt und der Rückstand zwischen Dichlormethan und Wasser verteilt. Die organische Phase wurde getrocknet und im Hochvakuum eingeengt. Der Rückstand wurde über eine präparative HPLC gereinigt (Waters Atlantis^{™} MS C₁₈ 5 µm, 30x100 mm, Acetonitril / (Wasser + 0,1 % TFA), Gradient 40 % auf 100 % Acetonitril in 25 Minuten). Man erhielt 382 mg (64 %) des gewünschten Produktes.
MS: *mlz* = 418,1 (M+H).

8-(4,5-Diphenyl-pyrimidin-2-ylamino)-oktansäure Trifluoracetat 106 mg (0,20 mmol) 8-(4,5-Diphenyl-pyrimidin-2-ylamino)-oktansäureethylester Trifluoracetat und 0,5 ml (1,00 mmol) 2 n Natriumhydroxidlösung wurden 5 Stunden bei Raumtemperatur gerührt. Anschließend wurde 1 ml (1,00 mmol) 1 n Natriumhydroxidlösung zugegeben und erneut 24 Stunden bei Raumtemperatur gerührt. Anschließend wurde mit Schwefelsäure auf pH 1 gestellt und mit Essigester extrahiert. Die organische Phase wurde getrocknet und im Vakuum eingeengt. Der Rückstand wurde mittels präparativer HPLC (Waters Atlantis^{™} MS C₁₈ 5 µm, 30x100 mm, Acetonitril / (Wasser + 0,1 % TFA), 10 % Acetonitril auf 95 % in 10 Minuten) getrennt. Man erhielt 12 mg (12 %) des gewünschten Produktes.
MS: *m*/*z* = 390,5 (M+H).

### Beispiel 4

### 7-(4,5-Diphenyl-pyrimidin-2-ylamino)-heptansäure Trifluoracetat

Aus 7-Amino-heptansäureethylester und 2-Chlor-4,5-diphenyl-pyrimidin erhielt man analog zu Beispiel 3 7-(4,5-Diphenyl-pyrimidin-2-ylamino)-heptansäure Trifluoracetat.

MS: m/z = 374,7 (M-H).

### Beispiel 5

### 6-(4,5-Diphenyl-pyrimidin-2-ylamino)-hexansäure Trifluoracetat

Aus 6-Amino-hexansäureethylester und 2-Chlor-4,5-diphenyl-pyrimidin erhielt man analog zu Beispiel 3 6-(4,5-Diphenyl-pyrimidin-2-ylamino)-hexansäure Trifluoracetat.

MS: m/z = 362,2 (M+H).

### Beispiel 6

### (R)-4-Amino-8-(4,5-diphenyl-pyrimidin-2-ylamino)-oktansäure Trifluoracetat

Zu einer Lösung von 135 mg (0,49 mmol) (R)-8-Amino-4-tert-butoxycarbonylamino-oktansäureethylester in 3,5 ml Dimethylformamid gab man 481 mg (1,48 mmol) Cäsiumcarbonat und rührte bei Raumtemperatur. Nach 60 Minuten wurde eine Lösung von 87 mg (0,33 mmol) 2-Chlor-4,5-diphenyl-pyrimidin in 2,5 ml Dimethylformamid zugegeben und 6 Stunden bei 90 °C gerührt. Das Lösungsmittel wurde eingeengt und der Rückstand zwischen Essigester und Wasser verteilt. Die organische Phase wurde getrocknet und im Hochvakuum eingeengt. Der Rückstand wurde über eine präparative HPLC gereinigt (Purosphere, Gradient Acetonitril / (Wasser + 0,5 % TFA), Gradient 20 % Acetonitril auf 100 % in 30 Minuten). Man erhielt 10 mg (R)-4-tert-Butoxycarbonylamino-8-(4,5-diphenyl-pyrimidin-2-ylamino)-oktansäureethylester. Das so gewonnene Produkt wurde 2 Stunden mit 50 % Trifluoressigsäure gerührt, mit Wasser versetzt und gefriergetrocknet. Man erhielt 12 mg (4 %) des gewünschten Produktes.

MS: *m*/*z* = 405,6 (M+H).

### Beispiel 7

### (S)-2-Amino-3-[4-(4,5-diphenyl-pyrimidin-2-ylamino)-cyclohexyl]-propionsäure Trifluoracetat

Zu einer Lösung von 161 mg (0,56 mmol) (S)-3-(4-Amino-cyclohexyl)-2-tert-butoxycarbonylamino-propionsäure in 3 ml Dimethylformamid gab man 550 mg (1,69 mmol) Cäsiumcarbonat und rührte bei Raumtemperatur. Nach 30 Minuten wurde eine Lösung von 100 mg (0,38 mmol) 2-Chlor-4,5-diphenyl-pyrimidin in 2 ml Dimethylformamid zugegeben und 6 Stunden bei 90 °C gerührt. Das Lösungsmittel wurde eingeengt und der Rückstand zwischen Essigester und Wasser verteilt. Die organische Phase wurde getrocknet und im Hochvakuum eingeengt. Der Rückstand wurde über eine präparative HPLC getrennt (Purosphere, Gradient Acetonitril / (Wasser + 0,5 % TFA), Gradient 20 % Acetonitril auf 100 % in 30 Minuten). Man erhielt 22 mg (S)-2-tert-Butoxycarbonylamino-3-[4-(4,5-diphenyl-pyrimidin-2-ylamino)-cyclohexyl]-propionsäure. Das so gewonnene Produkt wurde 16 Stunden mit 50 % Trifluoressigsäure in Acetonitril gerührt, mit Wasser versetzt und gefriergetrocknet. Man erhielt 17 mg (11 %) des gewünschten Produktes.

MS: m/z = 417,2 (M+H).

## Patentansprüche

1. Verbindungen der Formel I, 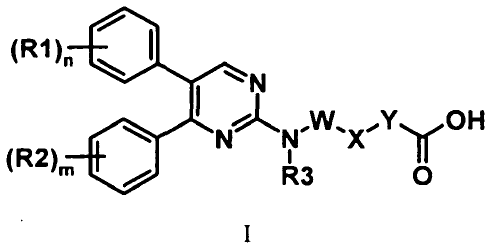 worin bedeuten
R1, R2 unabhängig voneinander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkl ein oder mehrfach mit F, Cl, Br oder CN substituiert sein kann;
n 0,1, 2, 3, 4 oder 5;
m 0, 1, 2, 3, 4 oder 5;
W Bindung, (C₁-C₇)-Alkylen, (C₂-C₇)-Alkenylen oder (C₂-C₇)-Alkinylen, wobei Alkylen, Alkenylen und Alkinylen ein oder mehrfach mit R4 substituiert sein können;
X Bindung, mono-, bi- oder tricyclischer (C₃-C₁₂)-Cycloalkylring, wobei der Cycloalkylring ein oder mehrfach mit R5 substituiert sein kann;
Y Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
R3 H oder (C₁-C₆)-Alkyl;
R4 NH₂, NH(C₁-C₄)-Alkyl, N[(C₁-C₄)-Alkyl]₂, F, Cl, Br, CN, OH, O-(C₁-C₆)-Alkyl, (C₁-C₆)-Alkyl, (C₂-C₆)-Alkenyl oder (C₂-C₆)-Alkinyl, wobei Alkl, Alkenyl und Alkinyl ein oder mehrfach mit F, Cl, Br oder CN substituiert sein können;
R5 F, Cl, Br, CN, (C₁-C₄)-Alkyl oder O-(C₁-C₄)-Alkyl, wobei Alkl ein oder mehrfach mit F, Cl, Br oder CN substituiert sein kann;
wobei W, X und Y nicht gleichzeitig die Bedeutung Bindung besitzen
und deren physiologisch verträgliche Salze.

2. Verbindungen der Formel I, gemäß Anspruch 1, **dadurch gekennzeichnet, dass** darin bedeuten
R1, R2 unabhängig voneinander (C₁-C₆)-Alkyl, O-(C₁-C₆)-Alkyl, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkyl ein oder mehrfach mit F, Cl, Br oder CN substituiert sein kann;
n 0, 1, 2, 3, 4 oder 5;
m 0, 1, 2, 3, 4 oder 5;
W Bindung, (C₁-C₇)-Alkylen, (C₂-C₇)-Alkenylen oder (C₂-C₇)-Alkinylen, wobei Alkylen, Alkenylen und Alkinylen ein oder mehrfach mit R4 substituiert sein können;
X Bindung, mono-, bi- oder tricyclischer (C₃-C₁₂)-Cycloalkylring, wobei der Cycloalkylring ein oder mehrfach mit R5 substituiert sein kann;
Y Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
R3 H oder (C₁-C₆)-Alkyl;
R4 NH₂, NH(C₁-C₄)-Alkyl, N[(C₁-C₄)-Alkyl]₂;
R5 F, Cl, Br, CN, (C₁-C₄)-Alkyl oder O-(C₁-C₄)-Alkyl, wobei Alkl ein oder mehrfach mit F, Cl, Br oder CN substituiert sein kann;
wobei W und X nicht gleichzeitig die Bedeutung Bindung besitzen;
und deren physiologisch verträgliche Salze.

3. Verbindungen der Formel I, gemäß Anspruch 1 oder 2, **dadurch gekennzeichnet, dass** darin bedeuten
R1, R2 unabhängig von einander (C₁-C₆)-Alkyl, O-(C₁-C₆)-Alkyl, OH, NH₂, NH(C₁-C₄)-Alkyl, N[(C₁-C₄)-Alkyl]₂, OH, CN, F, Cl, Br, O-Phenyl, CF₃, OCF₃ oder OCH₃, wobei Alkyl ein oder mehrfach mit F, Cl, Br oder CN substituiert sein kann;
n 0, 1, 2;
m 0, 1, 2;
W Bindung, (C₁-C₇)-Alkylen, (C₂-C₇)-Alkenylen oder (C₂-C₇)-Alkinylen, wobei Alkylen, Alkenylen und Alkinylen ein oder mehrfach mit R4 substituiert sein können;
X Bindung, mono-, bi- oder tricyclischer (C₃-C₁₂)-Cycloalkylring, wobei der Cycloalkylring ein oder mehrfach mit R5 substituiert sein kann;
Y Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
R3 H oder (C₁-C₆)-Alkyl;
R4 NH₂, NH(C₁-C₄)-Alkyl, N[(C₁-C₄)-Alkyl]₂;
R5 F, Cl, Br, CN, (C₁-C₄)-Alkyl oder O-(C₁-C₄)-Alkyl;
wobei W und X nicht gleichzeitig die Bedeutung Bindung besitzen;
und deren physiologisch verträgliche Salze.

4. Verbindungen der Formel I, gemäß einem oder mehreren der Ansprüche 1 bis 3, **dadurch gekennzeichnet, dass** darin bedeuten
R₁, R2 unabhängig voneinander (C₁-C₆)-Alkyl, O-(C₁-C₆)-Alkyl, OH, CN, F, Cl, Br, O-Phenyl, CF₃, OCF₃ oder OCH₃, wobei Alkyl ein oder mehrfach mit F, Cl, Br oder CN substituiert sein kann;
n 0;
m 0;
W Bindung, (C₁-C₇)-Alkylen; oder falls X eine Bindung ist W die Bedeutung (C₄-C₇)-Alkylen besitzt;
X Bindung, mono- oder bicyclischer (C₃-C₁₂)-Cycloalkylring, wobei der Cycloalkylring ein oder mehrfach mit R5 substituiert sein kann;
Y Bindung, (C₁-C₅)-Alkylen, (C₂-C₅)-Alkenylen oder (C₂-C₅)-Alkinylen, wobei Alkylen, Alkenylen und Alkinylen ein oder mehrfach mit R4 substituiert sein können;
R3 H oder (C₁-C₆)-Alkyl;
R4 NH₂, NH(C₁-C₄)-Alkyl, N[(C₁-C₄)-Alkyl]₂;
R5 F, Cl, Br, CN, (C₁-C₄)-Alkyl oder O-(C₁-C₄)-Alkyl;
wobei W und X nicht gleichzeitig die Bedeutung Bindung besitzen;
und deren physiologisch verträgliche Salze.

5. Verbindungen gemäß einem oder mehreren der Ansprüche 1 bis 4 zur Anwendung als Arzneimittel.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämische Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothclin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, CB 1-Rezeptor Antagonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

9. Verwendung der Verbindungen gemäß einem oder mehreren der Ansprüche 1 bis 4 zur Herstellung eines Medikamentes zur Blutzuckersenkung.

10. Verwendung der Verbindungen gemäß einem oder mehreren der Ansprüche 1 bis 4 zur Herstellung eines Medikamentes zur Behandlung des Diabetes.

11. Verwendung der Verbindungen gemäß einem oder mehreren der Ansprüche 1 bis 4 zur Herstellung eines Medikamentes zur Erhöhung der Insulinausschüttung.

12. Verfahren zur Herstellung eines Arzneimittels enthaltend eine oder mehrere der Verbindungen gemäß einem oder mehreren der Ansprüche 1 bis 4, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I, 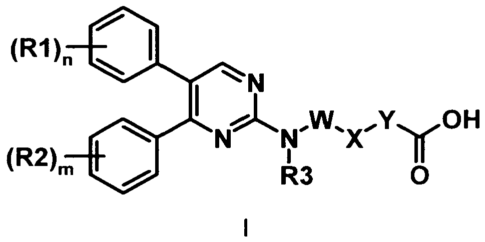 in which the meanings are
R1, R2 independently of one another (C₁-C₆)-alkyl, O-(C₁-C₆)-alkyl, NH₂, NH(C₁-C₄)-alkyl, N[(C₁-C₄)-alkyl]₂, OH, CN, F, Cl, Br, O-phenyl, CF₃, OCF₃ or OCH₃, where alkyl may be substituted one or more times by F, Cl, Br or CN;
n 0, 1, 2, 3, 4 or 5;
m 0, 1, 2, 3, 4 or 5;
W bond, (C₁-C₇)-alkylene, (C₂-C₇)-alkenylene or (C₂-C₇)-alkynylene, where alkylene, alkenylene and alkynylene may be substituted one or more times by R4;
X bond, mono-, bi- or tricyclic (C₃-C₁₂)-cycloalkyl ring, where the cycloalkyl ring may be substituted one or more times by R5;
Y bond, (C₁-C₅)-alkylene, (C₂-C₅)-alkenylene or (C₂-C₅)-alkynylene, where alkylene, alkenylene and alkynylene may be substituted one or more times by R4;
R3 H or (C₁-C₆)-alkyl;
R4 NH₂, NH(C₁-C₄)-alkyl, N[(C₁-C₄)-alkyl]₂, F, Cl, Br, CN, OH, O-(C₁-C₆)-alkyl, (C₁-C₆)-alkyl, (C₂-C₆)-alkenyl or (C₂-C₆)-alkynyl, where alkyl, alkenyl and alkynyl may be substituted one or more times by F, Cl, Br or CN;
R5 F, Cl, Br, CN, (C₁-C₄)-alkyl or O-(C₁-C₄)-alkyl, where alkyl may be substituted one or more times by F, Cl, Br or CN;
where W, X and Y do not simultaneously have the meaning of bond,
and the physiologically tolerated salts thereof.

2. A compound of the formula I as claimed in claim 1, in which the meanings are
R1, R2 independently of one another (C₁-C₆)-alkyl, O-(C₁-C₆)-alkyl, NH₂, NH(C₁-C₄)-alkyl, N[(C₁-C₄)-alkyl]₂, OH, CN, F, Cl, Br, O-phenyl, CF₃, OCF₃ or OCH₃, where alkyl may be substituted one or more times by F, Cl, Br or CN;
n 0,1, 2, 3, 4 or 5;
m 0, 1, 2, 3, 4 or 5;
W bond, (C₁-C₇)-alkylene, (C₂-C₇)-alkenylene or (C₂-C₇)-alkynylene, where alkylene, alkenylene and alkynylene may be substituted one or more times by R4;
X bond, mono-, bi- or tricyclic (C₃-C₁₂)-cycloalkyl ring, where the cycloalkyl ring may be substituted one or more times by R5;
Y bond, (C₁-C₅)-alkylene, (C₂-C₅)-alkenylene or (C₂-C₅)-alkynylene, where alkylene, alkenylene and alkynylene may be substituted one or more times by R4;
R3 H or (C₁-C₆)-alkyl;
R4 NH₂, NH(C₁-C₄)-alkyl, N[(C₁-C₄)-alkyl]₂;
R5 F, Cl, Br, CN, (C₁-C₄)-alkyl or O-(C₁-C₄)-alkyl, where alkyl may be substituted one or more times by F, Cl, Br or CN;
where W and X do not simultaneously have the meaning of bond;
and the physiologically tolerated salts thereof.

3. A compound of the formula I as claimed in claim 1 or 2, in which the meanings are
R1, R2 independently of one another (C₁-C₆)-alkyl, O-(C₁-C₆)-alkyl, OH, NH₂, NH(C₁-C₄)-alkyl, N[(C₁-C₄)-alkyl]₂, OH, CN, F, Cl, Br, O-phenyl, CF₃, OCF₃ or OCH₃, where alkyl may be subsituted one or more times by F, Cl, Br or CN;
n 0, 1, 2;
m 0, 1, 2;
W bond, (C₁-C₇)-alkylene; (C₂-C₇)-alkenylene or (C₂-C₇)-alkynylene, where alkylene, alkenylene and alkynylene may be substituted one or more times by R4;
X bond, mono-, bi- or tricyclic (C₃-C₁₂)-cycloalkyl ring, where the cycloalkyl ring may be substituted one or more times by R5;
Y bond, (C₁-C₅)-alkylene; (C₂-C₅)-alkenylene or (C₂-C₅)-alkynylene, where alkylene, alkenylene and alkynylene may be substituted one or more times by R4;
R3 H or (C₁-C₆)-alkyl;
R4 NH₂, NH(C₁-C₄)-alkyl, N[(C₁-C₄)-alkyl]₂;
R5 F, Cl, Br, (C₁-C₄)-alkyl or O-(C₁-C₄)-alkyl;
where W and X do not simultaneously have the meaning of bond;
and the physiologically tolerated salts thereof.

4. A compound of the formula I as claimed in one or more of claims 1 to 3, in which the meanings are
R1, R2 independently of one another (C₁-C₆)-alkyl, O-(C₁-C₆)-alkyl, OH, CN, F, Cl, Br, O-phenyl, CF₃, OCF₃ or OCH₃, where alkyl may be substituted one or more times by F, Cl, Br or CN
n 0;
m 0;
W bond, (C₁-C₇)-alkylene; or if X is a bond W has the meaning of (C₄-C₇)-alkylene;
X bond, mono- or bicyclic (C₃-C₁₂)-cycloalkyl ring, where the cycloalkyl ring may be substituted one or more times by R5;
Y bond, (C₁-C₅)-alkylene; (C₂-C₅)-alkenylene or (C₂-C₅)-alkynylene, where alkylene, alkenylene and alkynylene may be substituted one or more times by R4;
R3 H or (C₁-C₆)-alkyl;
R4 NH₂, NH(C₁-C₄)-alkyl, N[(C₁-C₄)-alkyl]₂;
R5 F, Cl, Br, CN, (C₁-C₄)-alkyl or O-(C₁-C₄)-alkyl;
where W and X do not simultaneously have the meaning of bond;
and the physiologically tolerated salts thereof.

5. A compound as claimed in one or more of claims 1 to 4 for use as pharmaceutical.

6. A pharmaceutical comprising one or more of the compounds as claimed in one or more of claims 1 to 4.

7. A pharmaceutical comprising one or more of the compounds as claimed in one or more of claims 1 to 4 and at least one further active ingredient.

8. A pharmaceutical as claimed in claim 7, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-bisphosphatase, modulators of glucose transporter 4 (GLUT4), inhibitors of glutamine-fructose-6-phosphate amidotransferase, inhibitors of dipeptidylpeptidase IV (DPP-IV), inhibitors of 11-beta-hydroxysteroid dehydrogenase 1, inhibitors of protein tyrosine phosphatase 1B, modulators of the sodium-dependent glucose transporter 1 or 2, inhibitors of hormone-sensitive lipase, inhibitors of acetyl-CoA carboxylase, inhibitors of phosphoenolpyruvate carboxykinase, inhibitors of glycoge synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists, lipase/amylase inhibitors, PPAR modulators, RXR modulators or TR-β agonists or amphetamines.

9. The use of the compounds as claimed in one or more of claims 1 to 4 for manufacturing a medicament for lowering blood glucose.

10. The use of the compounds as claimed in one or more of claims 1 to 4 for manufacturing a medicament for the treatment of diabetes.

11. The use of the compounds as claimed in one or more of claims 1 to 4 for manufacturing a medicament for increasing insulin release.

12. A process for manufacturing a pharmaceutical comprising one or more of the compounds as claimed in one or more of claims 1 to 4, which comprises mixing the active ingredient with a pharmaceutically suitable carrier and converting this mixture into a form suitable for administration.

## Revendications

1. Composés de formule I 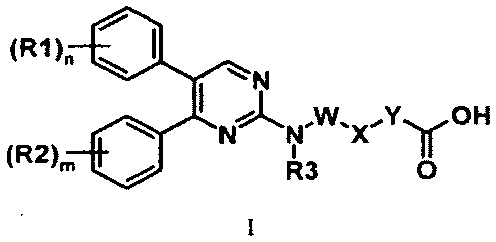 dans laquelle
R1, R2 signifient indépendamment l'un de l'autre alkyle en C₁-C₆, O-alkyle en C₁-C₆, NH₂, NH-alkyle en C₁-C₄, N[alkyle en C₁-C₄]₂, OH, CN, F, Cl, Br, O-phényle, CF₃, OCF₃ ou OCH₃ ; alkyle pouvant être substitué une ou plusieurs fois avec F, Cl, Br ou CN ;
n signifie 0, 1, 2, 3, 4 ou 5 ;
m signifie 0, 1, 2, 3, 4 ou 5 ;
W signifie une liaison, alkylène en C₁-C₇, alcénylène en C₂-C₇ ou alcynylène en C₂-C₇ ; alkylène, alcénylène et alcynylène pouvant être substitués une ou plusieurs fois avec R4 ;
X signifie une liaison, un cycle cycloalkyle en C₃-C₁₂ mono-, bi- ou tricyclique ; le cycle cycloalkyle pouvant être substitué une ou plusieurs fois avec R5 ;
Y signifie une liaison, alkylène en C₁-C₅, alcénylène en C₂-C₅ ou alcynylène en C₂-C₅ ; alkylène, alcénylène et alcynylène pouvant être substitués une ou plusieurs fois avec R4 ;
R3 signifie H ou alkyle en C₁-C₆
R4 signifie NH₂, NH-alkyle en C₁-C₄, N[alkyle en C₁-C₄]₂, F, Cl, Br, CN, OH, O-alkyle en C₁-C₆, alkyle en C₁-C₆, alcényle en C₂-C₆ ou alcynyle en C₂-C₆ ; alkyle, alcényle et alcynyle pouvant être substitués une ou plusieurs fois avec F, CI, Br ou CN ;
R5 signifie F, Cl, Br, CN, alkyle en C₁-C₄ ou O-alkyle en C₁-C₄ ; alkyle pouvant être substitué une ou plusieurs fois avec F, Cl, Br ou CN; W, X et Y n'ayant pas simultanément la signification liaison ;
et leurs sels physiologiquement compatibles.

2. Composés de formule 1 selon la revendication 1, **caractérisés en ce que**
R1, R2 signifient indépendamment l'un de l'autre alkyle en C₁-C₆, O-alkyle en C₁-C₆, NH₂, NH-alkyle en C₁-C₄, N[alkyle en C₁-C₄]₂, OH, CN, F, Cl, Br, O-phényle, CF₃, OCF₃ ou OCH₃ ; alkyle pouvant être substitué une ou plusieurs fois avec F, Cl, Br ou CN ;
n signifie 0, 1, 2, 3, 4 ou 5 ;
m signifie 0, 1, 2, 3, 4 ou 5 ;
W signifie une liaison, alkylène en C₁-C₇, alcénylène en C₂-C₇ ou alcynylène en C₂-C₇; alkylène, alcénylène et alcynylène pouvant être substitués une ou plusieurs fois avec R4 ;
X signifie une liaison, un cycle cycloalkyle en C₃-C₁₂ mono-, bi- ou tricyclique ; le cycle cycloalkyle pouvant être substitué une ou plusieurs fois avec R5 ;
Y signifie une liaison, alkylène en C₁-C₅, alcénylène en C₂-C₅ ou alcynylène en C₂-C₅; alkylène, alcénylène et alcynylène pouvant être substitués une ou plusieurs fois avec R4 ;
R3 signifie H ou alkyle en C₁-C_{6;}
R4 signifie NH₂, NH-alkyle en C₁-C₄, N[alkyle en C₁-C_{4]2} ;
R5 signifie F, CI, Br, CN, alkyle en C₁-C₄ ou O-alkyle en C₁-C₄ ; alkyle pouvant être substitué une ou plusieurs fois avec F, Cl, Br ou CN ; W et X n'ayant pas simultanément la signification liaison ;
et leurs sels physiologiquement compatibles.

3. Composés de formule I selon la revendication 1 ou 2, **caractérisés en ce que**
R1, R2 signifient indépendamment l'un de l'autre alkyle en C₁-C₆, O-alkyle en C₁-C₆, OH, NH₂, NH-alkyle en C₁-C₄, N[alkyle en C₁-C₄]₂, OH, CN, F, CI, Br, O-phényle, CF₃, OCF₃ ou OCH₃ ; alkyle pouvant être substitué une ou plusieurs fois avec F, CI, Br ou CN ;
n signifie 0, 1, 2 ;
m signifie 0, 1, 2 ;
W signifie une liaison, alkylène en C₁-C₇, alcénylène en C₂-C₇ ou alcynylène en C₂-C₇ ; alkylène, alcénylène et alcynylène pouvant être substitués une ou plusieurs fois avec R4 ;
X signifie une liaison, un cycle cycloalkyle en C₃-C₁₂ mono-, bi- ou tricyclique ; le cycle cycloalkyle pouvant être substitué une ou plusieurs fois avec R5 ;
Y signifie une liaison, alkylène en C₁-C₅, alcénylène en C₂-C₅ ou alcynylène en C₂-C₅ ; alkylène, alcénylène et alcynylène pouvant être substitués une ou plusieurs fois avec R4;
R3 signifie H ou alkyle en C₁-C₆ ;
R4 signifie NH₂, NH-alkyle en C₁-C₄, N[alkyle en C₁-C₄]₂ ;
R5 signifie F, CI, Br, CN, alkyle en C₁-C₄ ou O-alkyle en C₁-C₄ ;
W et X n'ayant pas simultanément la signification liaison ;
et leurs sels physiologiquement compatibles.

4. Composés de formule I selon une ou plusieurs des revendications 1 à 3, **caractérisés en ce que**
R1, R2 signifient indépendamment l'un de l'autre alkyle en C₁-C₆, O-alkyle en C₁-C₆, OH, CN, F, CI, Br, O-phényle, CF₃, OCF₃ ou OCH₃ ; alkyle pouvant être substitué une ou plusieurs fois avec F, Cl, Br ou CN ;
n signifie 0 ;
m signifie 0 ;
W signifie une liaison, alkylène en C₁-C₇ ; ou si X est une liaison, W a la signification alkylène en C₄-C₇ ;
X signifie une liaison, un cycle cycloalkyle en C₃-C₁₂ mono- ou bicyclique ; le cycle cycloalkyle pouvant être substitué une ou plusieurs fois avec R5 ;
Y signifie une liaison, alkylène en C₁-C₅, alcénylène en C₂-C₅ ou alcynylène en C₂-C₅ alkylène, alcénylène et alcynylène pouvant être substitués une ou plusieurs fois avec R4;
R3 signifie H ou alkyle en C₁-C_{6;}
R4 signifie NH₂, NH-alkyle en C₁-C₄, N[alkyle en C₁-C₄]₂ ;
R5 signifie F, Cl, Br, CN, alkyle en C₁-C₄ ou O-alkyle en C₁-C₄ ;
W et X n'ayant pas simultanément la signification liaison ;
et leurs sels physiologiquement compatibles.

5. Composés selon une ou plusieurs des revendications 1 à 4 pour une utilisation en tant que médicament.

6. Médicament contenant un ou plusieurs des composés selon une ou plusieurs des revendications 1 à 4.

7. Médicament contenant un ou plusieurs des composés selon une ou plusieurs des revendications 1 à 4 et au moins un autre agent actif.

8. Médicament selon la revendication 7, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de résorption du cholestérol, agonistes de PPAR gamma, agonistes de PPAR alpha, agonistes de PPAR alpha/gamma, agonistes de PPAR delta, fibrates, inhibiteurs de MTP, inhibiteurs de résorption d'acides biliaires, inhibiteurs de MTP, inhibiteurs de CETP, adsorbeurs polymères d'acides biliaires, inducteurs des récepteurs de LDL, inhibiteurs d'ACAT, antioxydants, inhibiteurs de lipoprotéine-lipase, inhibiteurs d'ATP-citrate-lyase, inhibiteurs de squalène synthétase, antagonistes de lipoprotéine (a), agonistes du récepteur HM74A, inhibiteurs de lipase, insulines, sulfonylurées, biguanides, méglitinides, thiazolidinedione, inhibiteurs d'α-glucosidase, agents actifs agissant sur le canal calcique des cellules bêta dépendant de l'ATP, inhibiteurs de la glycogène phosphorylase, antagonistes du récepteur du glucagon, activateurs de glucokinase, inhibiteurs de gluconéogénèse, inhibiteurs de fructose-1,6-diphosphatase, modulateurs du transporteur 4 de glucose, inhibiteurs de glutamine-fructose-6-phosphate-amidotransférase, inhibiteurs de dipeptidylpeptidase IV, inhibiteurs de 11-bêta-hydroxystéroïde-déshydrogénase 1, inhibiteurs de protéine-tyrosine-phosphatase 1 B, modulateurs du transporteur de glucose 1 ou 2 dépendant du sodium, inhibiteurs de la lipase sensible aux hormones, inhibiteurs de l'acétyl-CoA carboxylase, inhibiteurs de phosphoénolpyruvate-carboxykinase, inhibiteurs la glycogène synthase kinase-3 bêta, inhibiteurs de protéine kinase C bêta, antagonistes du récepteur d'endothéline A, inhibiteurs de I kappaB kinase, modulateurs du récepteur des glucocorticoïdes, agonistes de CART, agonistes de NPY, agonistes de MC4, agonistes d'orexine, agonistes d'H3, agonistes de TNF, agonistes de CRF, antagonistes de CRF BP, agonistes d'urocortine, agonistes de β3, antagonistes du récepteur de CB1, agonistes de MSH (hormone stimulant les mélanocytes), agonistes de CCK, inhibiteurs du recaptage de sérotonine, composés de sertonine et noradrénergiques mixtes, agonistes de 5HT, agonistes de bombésine, antagonistes de galanine, hormones de croissance, composés libérant des hormones de croissance, agonistes de TRH, modulateurs de protéine découplante 2 ou 3, agonistes de leptine, agonistes de DA, inhibiteurs de lipase/amylase, modulateurs de PPAR, modulateurs de RXR ou agonistes de TR-β ou amphétamines.

9. Utilisation des composés selon une ou plusieurs des revendications 1 à 4 pour la fabrication d'un médicament pour la diminution de la glycémie.

10. Utilisation des composés selon une ou plusieurs des revendications 1 à 4 pour la fabrication d'un médicament pour le traitement du diabète.

11. Utilisation des composés selon une ou plusieurs des revendications 1 à 4 pour la fabrication d'un médicament pour l'augmentation de la distribution de l'insuline.

12. Procédé de fabrication d'un médicament contenant un ou plusieurs des composés selon une ou plusieurs des revendications 1 à 4, **caractérisé en ce que** l'agent actif est mélangé avec un véhicule pharmaceutiquement approprié et ce mélange est mis sous une forme appropriée pour l'administration.
